# FASCICULE DE BREVET EUROPEEN

(11) **EP 4 225 401 B1**
(45) Date de publication et mention de la délivrance du brevet: **25.03.2026**
(21) Numéro de dépôt: 21783546.1
(22) Date de dépôt: 05.10.2021
(51) Int. Cl.: A61M 5/142, A61M 5/162, A61M 5/24, A61M 5/145

(54) **SYSTÈME ET DISPOSITIF DE DISTRIBUTION D'UN PRODUIT**
SYSTEM UND VORRICHTUNG ZUR AUSGABE EINES PRODUKTS
SYSTEM AND DEVICE FOR DISPENSING A PRODUCT

(30) Priorité: 05.10.2020 FR 2010167
(43) Date de publication de la demande: 16.08.2023
(73) Titulaire: NEMERA LA VERPILLIERE, 38290 La Verpillière (FR)
(72) Inventeur: TODESCO, Marc, 38230 TIGNIEU-JAMEYZIEU (FR); GRENOT, Gaëtan, 69004 LYON (FR)
(74) Mandataire: LLR
(86) Numéro de dépôt international: PCT/EP2021/077474
(87) Numéro de publication internationale: WO 2022/074018

(56) Documents cités:
- US-A1- 2002 004 641
- US-A1- 2009 234 323
- US-A1- 2013 237 922
- US-A1- 2015 313 798

## Description

L'invention concerne un système de distribution d'un produit, notamment un produit liquide destiné à être introduit dans un site d'un sujet. Par ailleurs, l'invention concerne un dispositif de distribution comprenant le système de distribution.

On connaît déjà dans l'état la technique, un dispositif de distribution de médicament comprenant un réservoir et une membrane obturant le réservoir. Afin de permettre la distribution de produit contenu dans le réservoir, la membrane est déformable depuis une position de stockage vers une position d'assemblage dans laquelle une aiguille provoque l'éclatement de la membrane. Une telle membrane est particulièrement fine, de l'ordre de 0,1 à 0,2 mm, et est ainsi difficile à fabriquer et à assembler car cela requiert beaucoup de précautions et un positionnement très précis de la membrane. En outre, la déformation de la membrane est difficile à obtenir de manière reproductible, compte tenu de sa fine épaisseur et du fait qu'elle doit être soumise à une pression différentielle relativement importante afin de garantir que celle-ci se déforme suffisamment pour entrer effectivement en contact avec l'aiguille et provoquer son éclatement. Des documents de l'état de la technique pertinents figurent sous les références US 2002/004641 A1, US 2009/0234323 A1, US 2013/0237922 A1 et US 2015/0313798 A1.

L'invention a notamment pour but de proposer un dispositif de distribution de produit dont la réalisation est simplifiée, tout en permettant de préserver la stérilité du produit contenu dans le réservoir, et ce jusqu'à la distribution du produit hors du réservoir.

A cet effet l'invention a notamment pour objet un dispositif de distribution d'un produit, comprenant un réservoir de produit doté d'un col délimitant une ouverture, une membrane fermant l'ouverture et comportant ainsi un côté interne orienté vers le réservoir et un côté externe opposé au côté interne, une bague de fixation de la membrane sur le col, la membrane étant bi-stable en ce qu'elle est configurée pour basculer d'une première position stable, dans laquelle la membrane est bombée dans un premier sens de telle sorte qu'elle fait saillie à l'intérieur du réservoir et obture le réservoir, à une deuxième position stable, dans laquelle la membrane est bombée dans un deuxième sens opposé au premier sens de façon à être percée par une aiguille disposée du côté externe de la membrane.

Ainsi, on propose d'utiliser une membrane bi-stable qui, lorsqu'elle passe de la première position stable à la deuxième position stable, passe par une position intermédiaire instable qui génère des contraintes dans la membrane et qui la poussent vers la deuxième position stable. Ainsi, la membrane « claque » de la première position stable dans la deuxième position stable, ce qui garantit le perçage de la membrane par l'aiguille. Grâce à cela, la reproductibilité de la déformation de la membrane bi-stable est augmentée car la membrane bi-stable n'est pas nécessairement fine et son fonctionnement ne dépend pas d'une capacité de gonflement incertaine. Par ailleurs, un tel dispositif de distribution est réalisé de manière particulièrement simple, puisqu'il suffit d'utiliser une bague de fixation de la membrane sur le col, tout en permettant de préserver la stérilité du réservoir et du produit contenu dans le réservoir, depuis l'assemblage du réservoir dans le dispositif de distribution jusqu'à la distribution du produit hors du réservoir, ainsi que durant et après le remplissage du réservoir. La réalisation du dispositif de distribution est d'autant plus simple que, grâce à la bague de fixation de la membrane sur le réservoir, le réservoir peut être un réservoir standard tel que ceux utilisés couramment dans le domaine pharmaceutique, sans requérir de fonctionnalité spécifique à la fixation de la membrane. Cette bague de fixation peut par exemple former une férule. Enfin, la membrane autorise un assemblage particulièrement simple en ce que, de préférence, son unique fonction est de basculer entre une position de stockage et d'assemblage correspondant à la première position stable, et une position percée correspondant à la deuxième position stable, sans autre fonction additionnelle.

Suivant d'autres caractéristiques optionnelles du dispositif de distribution prises seules ou en combinaison :
- Le dispositif de distribution comprend l'aiguille propre à percer la membrane lorsque la membrane est dans la deuxième position stable.
- Dans sa première position stable, la membrane obture le réservoir de telle sorte qu'elle bloque la distribution de produit.
- Dans sa deuxième position stable, la membrane est bombée dans le deuxième sens de telle sorte qu'elle fait saillie à l'extérieur du réservoir.
- La bague de fixation est en matériau thermoplastique ou en matériau métallique, de préférence en aluminium. Ainsi, le maintien de la membrane sur le réservoir est réalisé de manière particulièrement économique.
- Le réservoir est en verre ou en matériau thermoplastique. Ainsi, le réservoir est réalisé de manière particulièrement simple et économique. Il peut en particulier s'agir d'un réservoir en verre standard, couramment utilisé dans le domaine pharmaceutique et simple à stériliser.
- Le réservoir possède une forme de seringue dotée d'un col. Ainsi, le réservoir est réalisé de manière particulièrement simple. Selon une autre variante, le réservoir possède une forme de cartouche dotée d'un col. Selon encore une autre variante, le réservoir est formé d'une poche souple raccordée à un col plus rigide que la poche souple.
- L'aiguille est une aiguille de connexion à une tubulure de distribution de produit, de préférence une aiguille de connexion à un cathéter de distribution de produit. Alternativement, l'aiguille est une aiguille d'injection de produit, laquelle comporte une extrémité de perçage de la membrane et une extrémité opposée d'injection de produit dans le site d'un sujet.
- L'ouverture est fermée uniquement par la membrane. Ainsi, le dispositif de distribution est réalisé de manière particulièrement simple.
- La membrane est montée en appui direct ou indirect sur le col du réservoir, de préférence en appui direct. En particulier, elle est sertie directement sur le col du réservoir par la bague de fixation. Ainsi, le dispositif de distribution est réalisé de manière particulièrement simple.
- La membrane est bombée dans le premier sens de telle sorte qu'elle fait saillie dans le col du réservoir.
- La membrane présente une partie périphérique qui est comprimée axialement entre la bague de fixation et le col du réservoir. La direction axiale est définie en référence avec un axe central du réservoir. En d'autres termes, la partie périphérique de la membrane est prise en sandwich entre la bague de fixation et le col du réservoir. Il y a donc un contact, et de préférence un contact direct, entre la membrane et la bague de fixation d'une part, et entre la membrane et le col du réservoir d'autre part. Il s'agit d'une version particulièrement simple du dispositif de distribution, tout particulièrement dans le cas d'une prise en sandwich directe de la membrane entre la bague de fixation et le col du réservoir, sans pièce intermédiaire.
- La bague de fixation est fixée directement autour du col du réservoir, de préférence est sertie autour du col du réservoir. Ainsi, la fixation de la membrane est réalisée de manière particulièrement simple.
- Le produit est un produit liquide, de préférence destiné à être introduit dans un sujet. Par exemple, le produit est un médicament.
- La membrane est en matériau élastomère, de préférence à base de bromobutyl ou de chlorobutyl. Ainsi, la membrane peut être réalisée de manière simple, économique et sans effet chimique sur un produit contenu dans le réservoir. De plus ces matières sont avantageuses en termes de propriétés d'étanchéité et de compatibilité avec des produits médicaments ainsi qu'avec des processus de stérilisation.
- Le dispositif de distribution comporte l'aiguille en tant qu'aiguille de connexion à une tubulure de distribution de produit, l'aiguille étant configurée pour être distante de la membrane lorsque la membrane est dans la première position stable et pour traverser la membrane lorsque la membrane est dans la deuxième position stable. Ainsi, dans le cas où la membrane bascule dans sa deuxième position stable en début de distribution de produit, le produit ne se trouve en contact avec l'aiguille et la tubulure de distribution que lors de la distribution de produit. La stérilité du produit dans le réservoir est ainsi préservée jusqu'à la distribution du produit hors du réservoir.
- L'aiguille est configurée pour permettre la distribution de produit hors du réservoir à travers l'aiguille lorsqu'elle traverse la membrane dans la deuxième position stable. Ainsi, la distribution du produit est réalisée de manière particulièrement simple.
- L'aiguille est montée fixe par rapport au réservoir.
- L'aiguille est montée fixe sur le réservoir. Ainsi, le positionnement de l'aiguille par rapport au col du réservoir est particulièrement aisé et ne requiert pas d'assemblage complexe de pièce, de sorte que le perçage de la membrane est réalisé de manière simple et fiable, lorsque la membrane passe dans la deuxième position stable. On comprend que l'aiguille est de préférence montée indirectement sur le réservoir.
- Le dispositif de distribution comprend un organe de perçage comprenant :
   + un élément de réception de l'aiguille, auquel l'aiguille est rattachée, l'aiguille étant avantageusement emmanchée dans ledit élément de réception de l'aiguille et,
   + un élément de fixation sur la bague de fixation et/ou sur le col du réservoir.
   Ainsi, la fixation de l'élément de réception de l'aiguille et donc de l'aiguille sur le réservoir est réalisée de manière simple, tout en offrant un positionnement simple et fiable de l'aiguille par rapport au col du réservoir.
- L'organe de perçage est fixé sur le réservoir de telle sorte qu'un assemblage étanche à l'air extérieur est réalisé entre l'aiguille et la membrane. Ainsi, la stérilité du produit contenu dans le réservoir est davantage préservée.
- L'élément de réception de l'aiguille est fixé au-dessus de la bague de fixation de sorte que la bague de fixation comprend au moins une portion positionnée entre l'élément de réception de l'aiguille et la membrane suivant un axe central du réservoir.
- L'élément de réception de l'aiguille est propre à former avec la membrane, lorsqu'elle est bombée dans le premier sens, une chambre étanche à l'air extérieur.
- L'élément de fixation et/ou l'élément de réception de l'aiguille sont en matériau thermoplastique. Ainsi, l'organe de perçage est réalisé de manière simple et économique.
- L'organe de perçage comprend un joint qui se trouve en appui sur la bague de fixation. Avantageusement le joint est positionné entre l'élément de réception de l'aiguille et la bague de fixation et est en appui axial contre la bague de fixation.
- L'élément de fixation comporte des pattes de clipsage encliquetées sur la bague de fixation et/ou le col du réservoir. Ainsi, la fixation de l'aiguille sur le réservoir est réalisée de manière particulièrement simple. Par ailleurs, une fois l'assemblage réalisé, un démontage volontaire ou accidentel de l'aiguille vis-à-vis du réservoir est évité ou rendu impossible sans endommagement des différents éléments assemblés. Un fonctionnement correct de l'ensemble composé de la membrane et de l'aiguille est ainsi garanti.

Alternativement, l'élément de fixation présente la forme d'un collier de serrage, lequel comporte une patte de clipsage encliquetée dans une boucle. Ainsi, la fixation de l'aiguille sur le réservoir est réalisée de manière particulièrement simple. Par ailleurs, une fois l'assemblage réalisé, un démontage volontaire ou accidentel de l'aiguille vis-à-vis du réservoir est évité. Un fonctionnement correct de l'ensemble composé de la membrane et de l'aiguille est ainsi garanti. En outre, l'encliquetage est réalisé tangentiellement, ce qui permet d'éviter une compression trop importante susceptible de déformer la bague de fixation. Cela augmente par conséquent la précision du positionnement de l'aiguille par rapport au réservoir, et par conséquent le positionnement de l'aiguille par rapport à la membrane.
- L'élément de réception de l'aiguille est monobloc avec l'élément de fixation.
- Alternativement, l'élément de réception de l'aiguille est propre à être assemblé avec l'élément de fixation.
- L'élément de réception de l'aiguille et l'élément de fixation comprennent des moyens complémentaires d'assemblage de l'élément de réception de l'aiguille à l'élément de fixation. Avantageusement, l'élément de réception de l'aiguille comprend un élément circonférentiel, tel une rainure ou nervure, propre à coopérer avec un élément circonférentiel correspondant, tel une nervure ou rainure, de l'élément de fixation.
- L'élément de réception de l'aiguille comprend suivant un axe central du réservoir une première extrémité traversée par l'aiguille et par rapport à laquelle une extrémité de perçage de l'aiguille est en saillie et une deuxième extrémité, opposée à la première extrémité, recouverte par un élément de protection tel un film ou un capuchon d'isolation par rapport à un environnement extérieur.
- L'élément de réception de l'aiguille comprend un canal de distribution de produit débouchant au niveau de sa deuxième extrémité et s'étendant entre la deuxième extrémité et l'aiguille.
- L'aiguille s'étend à travers l'élément de réception de l'aiguille et comprend une première extrémité de perçage de la membrane et une deuxième extrémité propre à être reliée directement ou indirectement à une tubulure de distribution de produit.
- L'aiguille et le dispositif de distribution sont assemblés dans un boîtier de telle sorte que l'aiguille est comprise dans un organe de perçage comprenant un ou plusieurs éléments de fixation destinés à être en interaction avec le boîtier, afin que l'aiguille soit fixe par rapport au réservoir. Ainsi, on a un assemblage qui reste simple mais sans contraintes et lien d'assemblage direct entre l'aiguille et le réservoir ou la bague de fixation, ceci simplifiant leur production.
- La distance maximale entre la première position stable et la deuxième position stable est comprise entre 3 mm et 10 mm, de préférence est égale à 5 mm. Ainsi, le fonctionnement de la membrane est réalisé de manière compacte. On entend par « distance maximale entre la première position stable et la deuxième position stable » la longueur de débattement maximale entre les deux positions de la membrane. Dans le cas où la membrane bi-stable a une forme générale de disque bombé, on définit la « distance maximale entre la première position stable et la deuxième position stable » comme la distance parcourue par le centre de la membrane entre la première position stable et la deuxième position stable.

- L'extrémité de l'aiguille la plus proche de la membrane est axialement distante de la membrane lorsque la membrane est dans la première position stable, d'une valeur inférieure ou égale à la différence entre la distance maximale entre la première position stable et la deuxième position stable et l'épaisseur de la membrane, cette valeur étant de 2 à 7 mm, de préférence inférieure à 4 mm. Ainsi, il est garanti que l'aiguille perce la membrane dans la deuxième position stable.

- La membrane comprend une partie centrale déformable entre la première position stable et la deuxième position stable, la partie centrale présentant une épaisseur entre 0,3 mm et 2,5 mm, de préférence égale à 1 mm.
- La partie périphérique présente une épaisseur comprise entre 1 et 5 fois l'épaisseur de la partie centrale, de préférence entre 2 et 4 fois l'épaisseur de la partie centrale. Ainsi, la fixation de la membrane au moyen de la bague de fixation est facilitée.
- La membrane possède une forme de disque et présente un diamètre compris entre 7 mm et 20 mm, de préférence égal à 13 mm. Ainsi, on dispose d'une membrane compacte.
- La partie centrale possède une forme de disque et présente un diamètre compris entre 5 mm et 16 mm, de préférence égal à 9 mm. Ainsi, on dispose d'une membrane présentant une surface de contact faible avec le produit contenu dans le réservoir, ce qui améliore la stérilité du produit contenu dans le réservoir.
- La membrane est configurée pour basculer d'une première position stable à une deuxième position stable lorsque la pression différentielle entre le côté interne et le côté externe de la membrane dépasse un seuil prédéterminé. Ainsi, le basculement de la première position stable à la deuxième position stable peut être réalisé simplement en faisant varier la pression appliquée sur la membrane.
- Le seuil prédéterminé est entre 0,4 bar et 3 bar, de préférence entre 0,6 bar et 0,7 bar. Ainsi, la pression différentielle nécessaire pour faire basculer la membrane de la première position stable dans la deuxième position stable est relativement faible, et avantageusement plus faible que la pression différentielle qui serait nécessaire pour obtenir le gonflement d'une membrane en vue de son éclatement. Ainsi, le perçage de la membrane est obtenu sans nécessité d'appliquer une pression importante, ce qui est particulièrement avantageux lorsque l'énergie doit être économisée ou que le réservoir est dimensionné pour résister à une pression peu importante.
- Le dispositif de distribution comporte une pompe et un moteur électrique d'entraînement de la pompe, la pompe étant configurée pour faire basculer la membrane de la première position stable dans la deuxième position stable et pour distribuer du produit hors du réservoir à travers l'aiguille lorsque la membrane est dans la deuxième position stable. Ainsi, le fonctionnement de la membrane est réalisé de manière particulièrement simple, puisqu'aucun élément d'actionnement supplémentaire à ceux permettant la distribution n'est nécessaire.
- Le dispositif de distribution comporte une batterie d'alimentation du moteur électrique.
- La pompe est une pompe configurée pour comprimer le produit contenu dans le réservoir, comme par exemple une pompe entraînant un piston coulissant dans le réservoir. Ainsi, la pompe est réalisée de manière particulièrement simple. Alternativement, la pompe est une pompe péristaltique agissant sur la tubulure de distribution raccordée à l'aiguille de connexion. Ainsi, la pompe permet d'aspirer le produit hors du réservoir. Ainsi, la pompe est réalisée de manière particulièrement simple.
- La pompe est configurée de sorte à produire une pression différentielle au moins égale au seuil prédéterminé entre le côté interne et le côté externe de la membrane.
- Le dispositif de distribution comporte un système de détermination de la position de la membrane. Ainsi, le fonctionnement correct de la membrane peut être contrôlé, par exemple en contrôlant que la membrane n'a pas accidentellement pris sa deuxième position stable avant que la distribution ne soit souhaitée.
- Le système de détermination de la position de la membrane comprend un organe de mesure de l'intensité ou du couple du moteur électrique entraînant la pompe. Ainsi, on dispose d'un moyen de contrôle particulièrement simple et fiable du fonctionnement de la membrane. En effet, lorsque la membrane passe de la première position stable à la deuxième position stable, la pression varie fortement et rapidement, ce qui est détectable via l'organe de mesure.
- Le système de détermination de la position de la membrane comprend un capteur de pression configuré pour mesurer une pression du côté interne et/ou du côté externe de la membrane, de préférence configuré pour mesurer une pression différentielle entre le côté interne et le côté externe de la membrane. Ainsi, on dispose d'un moyen simple pour contrôler la pression, et par conséquent le fonctionnement de la membrane. En effet, lorsque la membrane passe de la première position stable à la deuxième position stable, la pression varie fortement et rapidement, ce qui est détectable via le capteur de pression.
- Le système de détermination de la position de la membrane comprend un capteur de détection de la position de la membrane, de préférence un capteur choisi parmi le groupe comprenant un capteur optique, un capteur électromagnétique, un capteur inductif, un capteur capacitif, un capteur à rebond, un capteur à ultrasons, un capteur de contact. Ainsi, on dispose d'un moyen simple et standard pour contrôler la position de la membrane.

L'invention concerne notamment également un système de distribution d'un produit comprenant un dispositif de distribution d'un produit tel que décrit précédemment, et une unité de distribution de produit.

Suivant d'autres caractéristiques optionnelles du système de distribution prises seules ou en combinaison :
- L'unité de distribution de produit comprend un dispositif d'insertion d'une aiguille permettant l'insertion et l'injection d'un produit à une profondeur réglable afin de permettre différents types d'injection.
- Le dispositif d'insertion comprend un support d'aiguille sur lequel est montée l'aiguille d'insertion, le support d'aiguille étant monté mobile entre différentes positions dont au moins une position d'insertion.
- Le dispositif d'insertion comprend un cathéter monté mobile par rapport à l'aiguille et un support de cathéter apte à déplacer le cathéter avec l'aiguille lors du passage du support d'aiguille se déplace vers au moins une position d'insertion, et à le désolidariser de l'aiguille de sorte que le cathéter reste verrouillé en mouvement lorsque le support d'aiguille se rétracte de l'au moins une position d'insertion.
- Le dispositif d'insertion comprend une aiguille d'insertion, qui est distincte de l'aiguille comprise dans le dispositif de distribution, et qui permet de réaliser une insertion dans un site.
- Le système de distribution est portatif. Ceci permet à l'utilisateur de se déplacer et de mener des activités simples tout en recevant le produit qui peut, par exemple, être un produit médical dans le cadre d'un traitement thérapeutique.

L'invention a encore notamment pour objet un kit d'assemblage pour un système de distribution d'un produit, comprenant un dispositif de distribution tel que décrit précédemment, et de préférence une unité de distribution de produit.

Suivant d'autres caractéristiques optionnelles du kit d'assemblage prises seules ou en combinaison :
- Le kit d'assemblage comprend un premier module comprenant le dispositif de distribution de produit et une unité de distribution d'un produit, et un deuxième module comprenant des moyens de réglage de l'unité de distribution de produit et, de façon optionnelle des moyens de contrôle de l'unité de distribution de produit.
- Le premier module est monté de façon amovible sur le deuxième module.
- Les moyens de réglage et/ou les moyens de contrôle sont en lien avec le dispositif de distribution de produit, préférentiellement en interaction avec le système de détermination de la position de la membrane.
- Les moyens de réglage comprennent une molette ou un ensemble vis-écrou accessibles par un utilisateur.
- Les moyens de réglage comprennent des moyens de commande électroniques.
- Les moyens de contrôle comprennent au moins un capteur de distance et/ou au moins un capteur de déplacement.
- L'au moins un capteur de distance et/ou l'au moins un capteur de déplacement est un capteur inductif, capacitif ou à base d'optique, d'ultrasons, de micro-ondes ou encore de type optoélectronique.
- Le kit d'assemblage comprend une pompe configurée pour faire basculer la membrane de la première position stable à la deuxième position stable et pour distribuer du produit hors du réservoir à travers l'aiguille de connexion lorsque la membrane est dans la deuxième position stable.
- La pompe est directement intégrée dans le premier module ou dans le second module.
- Un moteur peut être intégré au premier module ou au second module. Le moteur permet l'entrainement de la pompe selon un mode de réalisation. De même, de manière optionnelle, le moteur peut permettre l'actionnement et/ou l'entrainement de l'unité de distribution de produit. Préférentiellement, le moteur est un moteur électrique.
- Le premier module du kit d'assemblage correspond à une partie jetable, et le deuxième module correspond à une partie réutilisable.

Ainsi, on dispose d'un système de distribution économique, dans lequel les éléments coûteux et réutilisables, par exemple un moteur, sont intégrés dans une partie réutilisable, tout en préservant la sécurité, du fait que les éléments en contact avec le produit, par exemple le dispositif de distribution, ne sont pas réutilisables et sont regroupé dans la partie jetable, bien distincte de la partie réutilisable.

Le kit d'assemblage permet, par exemple, au personnel médical ou au patient d'avoir tous les éléments à disposition pour effectuer un traitement ou bien de réapprovisionner le personnel médical ou le patient avec une partie des éléments nécessaires au système de distribution d'un produit dans un site, notamment une ou des parties jetables.

### Brève description des figures

L'invention sera mieux comprise à la lecture de la description qui va suivre donnée uniquement à titre d'exemple et faite en se référant aux dessins annexés dans lesquels :
[Fig. 1] la figure 1 est une vue schématique en coupe d'un dispositif de distribution selon un premier mode de réalisation.
[Fig. 2] la figure 2 est une vue schématique en coupe d'un détail du dispositif de distribution selon le premier mode de réalisation, dans lequel la membrane est dans sa première position stable.
[Fig. 3] la figure 3 est une vue schématique en coupe d'un détail du dispositif de distribution selon le premier mode de réalisation, dans lequel la membrane est dans sa deuxième position stable.
[Fig. 4] la figure 4 est une vue schématique en perspective en coupe d'un détail d'un dispositif de distribution selon un deuxième mode de réalisation.
[Fig. 5] la figure 5 est une vue schématique en perspective d'un détail du dispositif de distribution selon le deuxième mode de réalisation.

### Description détaillée

Sur toutes les figures, les mêmes références se rapportent aux mêmes éléments. Les modes de réalisation suivants sont des exemples. Bien que la description se réfère à un ou plusieurs modes de réalisation, ceci ne signifie pas nécessairement que chaque référence concerne le même mode de réalisation, ou que les caractéristiques s'appliquent seulement à un seul mode de réalisation. De simples caractéristiques de différents modes de réalisation peuvent également être combinées pour fournir d'autres modes de réalisation.

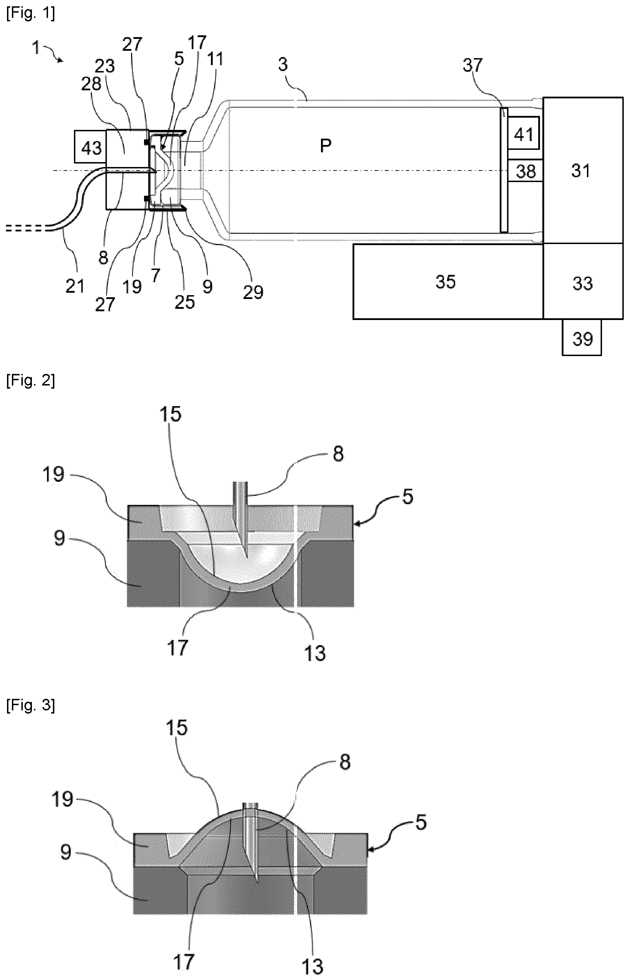

On a représenté sur la figure 1 un dispositif de distribution selon un premier mode de réalisation, désigné par la référence générale 1.

Le dispositif de distribution de produit 1 comporte un réservoir 3 de produit P, une membrane 5, une bague de fixation 7 et une aiguille 8. Le dispositif de distribution 1 est portatif.

Le réservoir 3 est doté d'un col 9 délimitant une ouverture 11. Plus précisément dans cet exemple, le réservoir 3 possède une forme de seringue dotée du col 9. Le réservoir 3 est en verre ou en matériau thermoplastique. Le produit P contenu dans le réservoir 3 est dans cet exemple un produit liquide, de préférence destiné à être introduit dans un sujet, par exemple un patient humain. Par exemple, le produit P est un médicament. Dans une variante non représentée, le réservoir possède une forme de cartouche dotée d'un col. Dans une autre variante non représentée, le réservoir comporte une poche souple raccordée à un col plus rigide que la poche souple, lequel permet la fixation de la membrane via la bague de fixation 7 sur le col.

Les produits, notamment pharmaceutiques, susceptibles d'être utilisés par le dispositif de distribution sont par exemple des formulations contenant au moins un principe actif tel que les peptides, les protéines, les hormones, les principes actifs d'origine biologique, les principes actifs à base de nucléotides, comme par exemple les ADN, les ARN ou les oligonucleotides, les principes actifs avec un poids moléculaire jusqu'à 1500 Da, les polysaccharides, les vaccins, les enzymes, les anticorps, les formules nutritionnelles et d'autres substances ou leur mélange.

Les produits, notamment pharmaceutiques, susceptibles d'être utilisés par le dispositif de distribution peuvent être utilisés pour le traitement et/ou la prévention de des diabètes, des thromboses, des maladies cardio-vasculaires, comme le syndrome coronarien, l'angine, l'infarctus du myocarde, des cancers, de la dégénérescence maculaire, des inflammations, des athéroscléroses et/ou des arthrites rhumatoïdes.

Ces principes actifs peuvent être, sans s'y limiter, les insulines, les analogues de l'insuline tels que l'insuline lispro ou l'insuline glargine, les dérivés de l'insuline, le C-peptide, les agonistes du récepteur GLP-1 tels que le dulaglutide ou le liraglutide, le glucagon, les analogues du glucagon, les dérivés du glucagon, les polypeptides inhibiteur gastrique (GIP), les analogues du GIP, les dérivés du GIP, les analogues de l'oxyntomoduline, les dérivés de l'oxyntomoduline, les anticorps thérapeutiques, tel que les anticorps monoclonaux et tout agent thérapeutique pouvant être délivré par le dispositif ci-dessus ainsi que un sel et/ou un solvate et/ou un hydrate pharmaceutiquement acceptable des principes actifs listés ci-dessus. Le médicament tel qu'il est utilisé dans le dispositif peut être formulé avec un ou plusieurs excipients.

En particulier le produit peut comprendre au moins un peptide pour le traitement et/ou la prévention d'un diabète.

En particulier le produit peut comprendre au moins une insuline humaine ou un dérivé ou un analogue d'insuline comme par exemple, le glucagon-like peptide (GLP-1) ou un analogue ou un dérivé du GLP-1, ou l'exedin-3, exedin-4 ou un de leurs analogues ou dérivés, ou les Gly(A21), Arg(B31), Arg(B32) insulines humaines; les Lys(B3), Glu(B29) insulines humaines; les Lys(B28), Pro(B29) insulines humaines, les Asp(B28) insulines humaines, les B29-N-myristoyl-des(B30) insulines humaines; B29-N-palmitoyl-des(B30) insulines humaines; B29-N-myristoyl insulines humaines; B29-N-palmitoyl insulines humaines; B28-N-myristoyl LysB28ProB29 insulines humaines; B28-N-palmitoyl-LysB28ProB29 insulines humaines; B30-N-myristoyl-ThrB29LysB30 insulines humaines; B30-N-palmitoyl- ThrB29LysB30 insulines humaines; B29-N-(N-palmitoyl-Y-glutamyl)-des(B30) insulines humaines; B29-N-(N-lithocholyl-Y-glutamyl)-des(B30) insulines humaines; B29-N-(ω-carboxyheptadecanoyl)-des(B30) insulines humaines and B29-N-(ω-carboxyheptadecanoyl) insulines humaines et/ou un sel et/ou un solvate et/ou un hydrate pharmaceutiquement acceptable des insulines listées ci-dessus.

En particulier le produit peut comprendre au moins une hormone comme par exemple, les hormones hypophysaires ou hypothalamiques comme par exemple les gonadotropines (follitropines, lutropines, choriongonadotropines, ménotropines), somatropines ou somatotropines, desmopressines, terlipressines, gonadorélines, triptorélines, leuprorélines, busérélines, nafarélines, gosérélines et/ou un sel et/ou un solvate et/ou un hydrate pharmaceutiquement acceptable des hormones listées ci-dessus.

En particulier le produit peut comprendre au moins un polysaccharide comme par exemple un glucosaminoglycane, un acide hyaluronique, une héparine, une héparine de faible poids moléculaire ou un dérivé de l'héparine, un polysaccharide sulfaté ou polysulfaté, et/ou un sel et/ou un solvate et/ou un hydrate pharmaceutiquement acceptable des polysaccharides listés ci-dessus.

Les sels pharmaceutiquement acceptables sont par exemple les sels d'addition acide, comme par exemple les sels de HCl ou de HBr, et les sels basiques, comme par exemple les sels composés d'un cation alcalin comme Na+, K+, ou Ca2+, ou un ion ammonium du type N+(R1)(R2)(R3)(R4),où R1, R2, R3 et R4 représentent indépendamment les uns des autres : un atome d'hydrogène, un groupement (C1-C6 ) alkyle optionnellement substitué, un groupement (C2-C6) alcényle optionnellement substitué, un groupement (C6-C10) aryle optionnellement substitué, ou un groupement (C6-C10) hétéroaryle optionnellement substitué. D'autres exemples de sels pharmaceutiquement acceptables sont bien connus de l'homme de l'art.

La bague de fixation 7 sert à la fixation de la membrane 5 sur le col 9. Dans cet exemple, la bague de fixation 7 est fixée directement autour du col 9 du réservoir 3. Plus précisément, la bague de fixation 7 est sertie autour du col 9 du réservoir 3, et possède un diamètre resserré à son extrémité distante de la membrane de manière à coopérer avec le col 9 et ainsi empêcher son extraction hors du col 9. La bague de fixation 7 est en matériau thermoplastique ou en matériau métallique, de préférence en aluminium.

La membrane 5 ferme l'ouverture 11 et comporte ainsi un côté interne 13 orienté vers le réservoir 3 et un côté externe 15 opposé au côté interne 13. La membrane 5 est une membrane bi-stable. Ainsi, la membrane 5 est configurée pour basculer d'une première position stable représentée en détail sur la figure 2, dans laquelle la membrane 5 est bombée dans un premier sens de telle sorte que la membrane 5 fait saillie à l'intérieur du réservoir 3 et obture le réservoir 3, à une deuxième position stable représentée en détail sur la figure 3, dans laquelle la membrane 5 est bombée dans un deuxième sens opposé au premier sens. Plus précisément, la membrane 5 bascule de la première position stable à la deuxième position stable lorsque la pression différentielle entre le côté interne 13 et le côté externe 15 de la membrane 5 dépasse un seuil prédéterminé. Le seuil prédéterminé est entre 0,4 bar et 3 bar, de préférence entre 0,6 bar et 0,7 bar. Ainsi dans cet exemple, dans sa première position stable, la membrane 5 obture le réservoir 3 de telle sorte qu'elle bloque la distribution de produit P, et dans sa deuxième position stable, la membrane 5 est bombée dans le deuxième sens de telle sorte qu'elle fait saillie à l'extérieur du réservoir 3. Dans cet exemple, l'ouverture 11 est fermée uniquement par la membrane 5. La membrane 5 est montée en appui direct sur le col 9 du réservoir 3, la membrane 5 étant sertie directement sur le col 9 du réservoir 3 par la bague de fixation 7. La membrane 5 possède une forme de disque et présente un diamètre compris entre 7 mm et 20 mm, de préférence égal à 13 mm. Comme représenté sur la figure 2, lors de l'assemblage et du stockage, la membrane 5 est bombée dans le premier sens de telle sorte qu'elle fait saillie dans le col 9 du réservoir 3, à travers l'ouverture 11. La membrane 5 comprend une partie périphérique 19 et une partie centrale 17 déformable entre la première position stable et la deuxième position stable. La partie centrale 17 présente une épaisseur entre 0,3 mm et 2,5 mm, de préférence égale à 1 mm. La partie centrale 17 possède une forme de disque et présente un diamètre compris entre 5 mm et 16 mm, de préférence égal à 9 mm. La partie périphérique 19 est fixe, du fait qu'elle est comprimée axialement entre la bague de fixation 7 et le col 9 du réservoir 3. Dans un état non comprimé, la partie périphérique 19 présente une épaisseur comprise entre 1 et 5 fois l'épaisseur de la partie centrale 17, de préférence entre 3 et 4 fois l'épaisseur de la partie centrale 17. La distance maximale entre la première position stable et la deuxième position stable est comprise entre 3 mm et 10 mm, de préférence est égale à 5 mm. Cette distance maximale correspond à la distance entre le centre de la membrane 5 situé sur la partie centrale 17 bombée dans le premier sens dans la première position stable et le centre de la membrane 5 situé sur la partie centrale 17 bombée dans le deuxième sens dans la deuxième position stable. La membrane 5 est en matériau élastomère, de préférence à base de bromobutyl ou de chlorobutyl. La membrane 5, lorsqu'elle bascule dans sa deuxième position stable, est bombée dans le deuxième sens de façon à être percée par l'aiguille 8 disposée du côté externe 15 de la membrane 5.

Dans cet exemple, l'aiguille 8 est une aiguille de connexion à une tubulure de distribution 21 de produit P, de préférence une aiguille de connexion à un cathéter de distribution de produit. L'aiguille 8 est en matériau métallique, par exemple en acier inoxydable. Selon une variante, l'aiguille 8 est en matériau plastique, de préférence thermoplastique. Par exemple, l'aiguille 8 est reliée à un dispositif d'insertion via la tubulure de distribution 21. Ce dispositif d'insertion comprend une aiguille d'insertion permettant l'insertion dans un site du produit P compris dans le réservoir 3 et distribué via l'aiguille 8 lorsque la membrane 5 est dans sa deuxième position stable. Avantageusement, ce dispositif d'insertion peut aussi comprendre un support d'aiguille sur lequel est montée l'aiguille d'insertion, le support d'aiguille étant monté mobile entre différentes positions dont au moins une position d'insertion. Préférentiellement, le dispositif d'insertion comprend un cathéter monté mobile par rapport à l'aiguille d'insertion et un support de cathéter apte à déplacer le cathéter avec l'aiguille d'insertion lors du passage du support d'aiguille se déplace vers au moins une position d'insertion, et à le désolidariser de l'aiguille d'insertion de sorte que le cathéter reste verrouillé en mouvement lorsque le support d'aiguille se rétracte de l'au moins une position d'insertion.

Selon une variante non représentée, l'aiguille est une aiguille d'injection de produit, laquelle comporte une extrémité de perçage de la membrane 5 et une extrémité opposée d'injection de produit dans un site d'un sujet.

L'aiguille 8 est distante de la membrane 5 lorsque la membrane 5 est dans la première position stable et traverse la membrane 5 lorsque la membrane 5 est dans la deuxième position stable. Ainsi, l'aiguille 8 permet la distribution du produit P hors du réservoir 3 à travers l'aiguille 8 lorsqu'elle traverse la membrane 5 dans la deuxième position stable. L'aiguille 8 est montée fixe par rapport au réservoir 3. Dans cet exemple, l'aiguille 8 est montée fixe sur le réservoir 3. Pour cela, l'aiguille 8 est emmanchée dans un organe de perçage 23, lequel comporte un élément de fixation 25 sur la bague de fixation 7 et/ou sur le col 9 du réservoir 3. L'organe de perçage 23 est également appelé organe de fixation et comprend l'élément de fixation 25 et un élément de réception 28 de l'aiguille 8 dans lequel l'aiguille 8 est rattachée, avantageusement emmanchée. L'élément de réception 28 de l'aiguille 8 est fixé au-dessus de la bague de fixation 7 de sorte que la bague de fixation 7 comprend au moins une portion positionnée entre l'élément de réception 28 de l'aiguille 8 et la membrane 5 suivant un axe central du réservoir 3. L'aiguille 8 s'étend à travers l'élément de réception 28 de l'aiguille 8 et comprend une première extrémité de perçage de la membrane 5 et une deuxième extrémité propre à être reliée directement ou indirectement à la tubulure de distribution 21 de produit P.

L'élément de fixation 25 et/ou l'élément de réception 28 de l'aiguille 8 sont en matériau thermoplastique. Dans cet exemple, l'organe de perçage 23 est en matériau thermoplastique. L'organe de perçage 23 est fixé sur le réservoir 3 de telle sorte qu'un assemblage étanche à l'air extérieur est réalisé entre l'aiguille 8 et la membrane 5. Ainsi, l'élément de réception 28 de l'aiguille 8 est propre à former avec la membrane 5, lorsqu'elle est bombée dans le premier sens, une chambre étanche à l'air extérieur. Pour cela, l'organe de perçage 23 comprend un joint 27 qui se trouve en appui sur la bague de fixation 7. Dans cet exemple, le joint 27 est positionné entre l'élément de réception 28 de l'aiguille 8 et la bague de fixation 7 et est en appui axial contre la bague de fixation 7.

L'élément de réception 28 de l'aiguille 8 comprend suivant un axe central du réservoir une première extrémité traversée par l'aiguille 8 et par rapport à laquelle une extrémité de perçage de l'aiguille 8 est en saillie et une deuxième extrémité, opposée à la première extrémité. L'élément de réception 28 de l'aiguille 8 comprend un canal de distribution de produit P débouchant au niveau de sa deuxième extrémité et s'étendant entre la deuxième extrémité et l'aiguille 8. Selon une variante, la deuxième extrémité de l'élément de réception 28 de l'aiguille 8 est recouverte par un élément de protection tel un film ou un capuchon d'isolation par rapport à un environnement extérieur. L'élément de protection est retiré avant de relier directement ou indirectement la deuxième extrémité de l'aiguille 8 à la tubulure de distribution 21 de produit P.

Dans cet exemple, le joint 27 est annulaire. L'élément de fixation 25 comporte des pattes de clipsage 29 encliquetées sur la bague de fixation 7, autour du col 9, et/ou sur le col 9 du réservoir 3. Dans cet exemple, l'élément de fixation 25 est venu de matière avec l'organe de perçage 23. En d'autres termes, l'élément de réception de l'aiguille 8 est monobloc avec l'élément de fixation. Alternativement, selon une variante, l'élément de réception de l'aiguille 8 est propre à être assemblé avec l'élément de fixation. Selon cette variante, l'élément de réception de l'aiguille 8 et l'élément de fixation comprennent des moyens complémentaires d'assemblage de l'élément de réception de l'aiguille 8 à l'élément de fixation. Avantageusement, selon cette variante, l'élément de réception de l'aiguille 8 comprend un élément circonférentiel, tel une rainure ou une nervure, propre à coopérer avec un élément circonférentiel correspondant, tel une nervure ou une rainure, de l'élément de fixation.

L'aiguille 8 est disposée de sorte à traverser la membrane 5 lorsque la membrane 5 est dans la deuxième position stable. Ainsi, le dispositif de distribution 1 comprend l'aiguille 8 propre à percer la membrane 5 lorsque la membrane 5 est dans la deuxième position stable. Pour cela, l'extrémité de l'aiguille 8 la plus proche de la membrane 5 est axialement distante de la membrane 5, plus précisément en regard du côté externe 15 de la membrane 5, lorsque la membrane 5 est dans la première position stable. Cette distance est d'une valeur inférieure ou égale à la différence entre la distance maximale entre la première position stable et la deuxième position stable et l'épaisseur de la membrane 5, cette valeur étant comprise entre 2 et 7 mm, de préférence inférieure à 4 mm.

Afin de permettre la distribution de produit P, le dispositif de distribution 1 comporte dans cet exemple une pompe 31, un moteur électrique 33 d'entraînement de la pompe 31 et une batterie 35 d'alimentation du moteur électrique 33.

La pompe 31 est configurée pour faire basculer la membrane 5 de la première position stable dans la deuxième position stable et pour distribuer du produit hors du réservoir 3 à travers l'aiguille 8 lorsque la membrane 5 est dans la deuxième position stable. Dans cet exemple, la pompe 31 est une pompe configurée pour comprimer le produit P contenu dans le réservoir 3. Par exemple, la pompe 31 entraîne un piston 37 coulissant dans le réservoir 3. Selon une deuxième variante non représentée, la pompe 31 est une pompe péristaltique agissant sur la tubulure de distribution 21 raccordée à l'aiguille 8.

La pompe 31 est configurée de sorte à produire une pression différentielle au moins égale au seuil prédéterminé entre le côté interne 13 et le côté externe 15 de la membrane 5. Grâce à cela, la pompe 31 assure à la fois la mise en communication de la tubulure de distribution 21 et du réservoir 3 via l'aiguille 8 lorsque la membrane 5 bascule de la première position stable dans la deuxième position stable, puis la distribution effective du produit P via la tubulure de distribution 21.

Dans le but de contrôler le fonctionnement de la membrane 5, le dispositif de distribution 1 comporte un système de détermination de la position de la membrane 5. Comme représenté sur la figure 1, le système de détermination de la position de la membrane 5 comporte un organe de mesure 39 de l'intensité ou du couple du moteur électrique 33 entraînant la pompe 31. Le système de détermination de la position de la membrane comprend également un capteur de pression 41 configuré pour mesurer une pression du côté interne 13 et/ou du côté externe 15 de la membrane 5, de préférence configuré pour mesurer une pression différentielle entre le côté interne 13 et le côté externe 15 de la membrane 5. Le système de détermination de la position de la membrane 5 comprend en outre un capteur de détection 43 de la position de la membrane 5, de préférence un capteur choisi parmi le groupe comprenant un capteur optique, un capteur électromagnétique, un capteur inductif, un capteur capacitif, un capteur à rebond, un capteur à ultrasons, un capteur de contact.

Afin d'assembler un système de distribution comprenant le dispositif de distribution 1, on réalise un premier module comprenant le réservoir 3 contenant du produit P, la bague de fixation 7, la membrane 5, l'aiguille 8, une première partie du système de détermination de la position de la membrane 5 et une unité de distribution de produit (non représentée). On réalise aussi un deuxième module comprenant la pompe 31 et le moteur électrique 33 d'entraînement de la pompe 31, une seconde partie du système de détermination de la position de la membrane 5 ainsi que des moyens de réglage (non représentés) de l'unité de distribution de produit, la pompe 31 étant configurée pour basculer la membrane 5 de la première position stable dans la deuxième position stable et pour distribuer du produit P hors du réservoir 3 à travers l'aiguille 8 lorsque la membrane 5 est dans la deuxième position stable. Le premier module et le deuxième module composent ainsi un kit d'assemblage, le premier module étant à usage unique et le deuxième module étant réutilisable dans la configuration décrite. Par kit d'assemblage pour un système de distribution d'un produit, il faut comprendre qu'un tel kit d'assemblage comporte des éléments disposés séparément, ici le premier module et le deuxième module, lesquels sont destinés à être assemblés ultérieurement pour former un système de distribution de produit. Ainsi, le kit d'assemblage pour un système de distribution d'un produit comporte un dispositif de distribution 1 et une unité de distribution de produit.

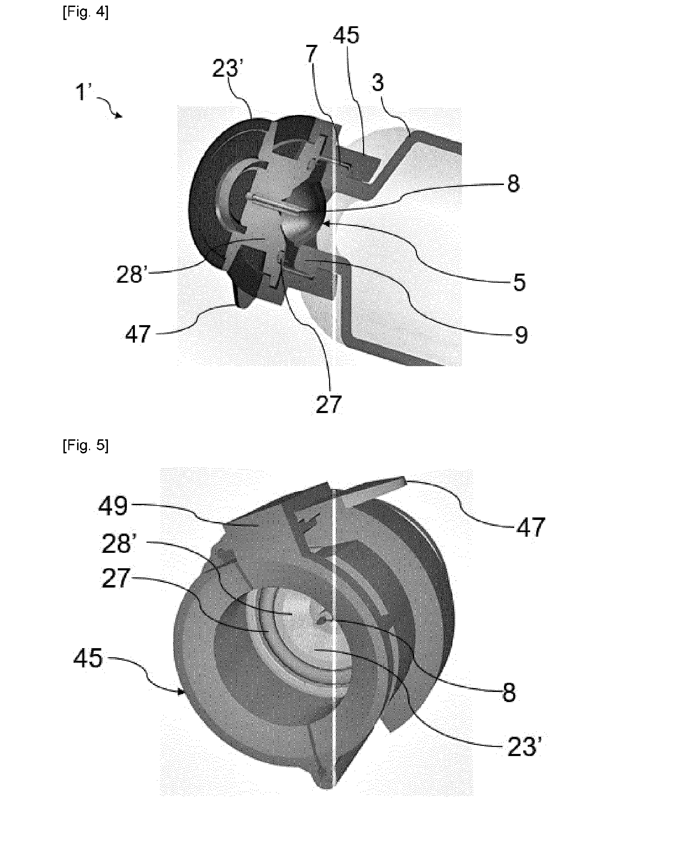

On a représenté sur les figures 4 et 5 un détail d'un dispositif de distribution 1' selon un deuxième mode de réalisation. Le dispositif de distribution 1' selon ce deuxième mode de réalisation se distingue du dispositif de distribution 1 selon le premier mode de réalisation en ce que l'organe de perçage 23', également appelé organe de fixation, comporte un élément de fixation 45 structurellement différent de l'élément de fixation 25 du premier mode de réalisation, mais remplissant la même fonction de fixation de l'aiguille 8 sur le réservoir 3 afin de la positionner par rapport à la membrane 5. Dans ce deuxième mode de réalisation, l'élément de fixation 45 présente la forme d'un collier de serrage, lequel comporte une patte de clipsage 47 encliquetée dans une boucle 49. L'encliquetage est ainsi réalisé tangentiellement. L'élément de fixation 45 est par exemple en matériau thermoplastique. Lors de l'assemblage, l'organe de perçage 23' est prépositionné par rapport au réservoir 3, et l'élément de fixation 45 est ensuite rapporté et serré à la fois sur l'organe de perçage 23' et sur la bague de fixation 7 et/ou sur le col 9 du réservoir 3. Le serrage est réalisé par encliquetage de la patte de clipsage 47 dans la boucle 49. Dans ce mode de réalisation, l'organe de perçage 23 comprend également un joint 27 annulaire, lequel se trouve en appui sur la bague de fixation 7 lorsque le dispositif de distribution 1' est à l'état assemblé. Dans cet exemple, l'élément de réception 28' de l'aiguille 8 est propre à être assemblé avec l'élément de fixation 45. Ainsi, l'élément de réception 28' de l'aiguille 8 et l'élément de fixation 45 comprennent des moyens complémentaires d'assemblage de l'élément de réception 28' de l'aiguille 8 à l'élément de fixation 45. Avantageusement, l'élément de réception 28' de l'aiguille 8 comprend un élément circonférentiel, tel une rainure ou une nervure, propre à coopérer avec un élément circonférentiel correspondant, tel une nervure ou une rainure, de l'élément de fixation 45.

L'élément de réception 28' de l'aiguille 8 comprend suivant un axe central du réservoir une première extrémité traversée par l'aiguille 8 et par rapport à laquelle une extrémité de perçage de l'aiguille 8 est en saillie et une deuxième extrémité, opposée à la première extrémité, recouverte par un élément de protection tel un film ou un capuchon d'isolation par rapport à un environnement extérieur. L'élément de réception 28' de l'aiguille 8 comprend un canal de distribution de produit P débouchant au niveau de sa deuxième extrémité et s'étendant entre la deuxième extrémité et l'aiguille 8.

Dans le mode de réalisation présenté sur les figures, l'aiguille est une aiguille biseau dont l'extrémité de perçage de la membrane comprend une ouverture orientée vers la membrane et propre à être traversée par le produit. Lors de la distribution de produit, le produit est alors propre à passer à travers l'aiguille depuis l'extrémité de perçage vers une extrémité de distribution opposée à l'extrémité de perçage suivant un axe longitudinal de l'aiguille. En variante, l'aiguille est une aiguille de type « pencil point needle » comprenant entre l'extrémité de perçage de la membrane et une extrémité de distribution opposée à l'extrémité de perçage suivant un axe longitudinal de l'aiguille, une ouverture latérale propre à être traversée par le produit. Lors de la distribution de produit, le produit est alors propre à passer à travers l'aiguille depuis l'ouverture latérale vers l'extrémité de distribution.

L'invention n'est pas limitée aux modes de réalisation présentés et d'autres modes de réalisation apparaîtront clairement à l'homme du métier. Il est notamment possible de combiner les modes de réalisation entre eux.

### Liste de références

1, 1' : dispositif de distribution
3 : réservoir
5 : membrane
7 : bague de fixation
8 : aiguille
9 : col
11 : ouverture
13 : côté interne
15 : côté externe
17 : partie centrale
19 : partie périphérique
21 : tubulure de distribution
23, 23' : organe de perçage
25 : élément de fixation
27 : joint
28, 28' : élément de réception
29 : patte de clipsage
31 : pompe
33 : moteur électrique
35 : batterie
37 : piston
38 : tige
39 : organe de mesure
41 : capteur de pression
43 : capteur de détection
45 : élément de fixation
47 : patte de clipsage
49 : boucle
P : produit

## Revendications

1. Dispositif de distribution (1, 1') d'un produit (P), comprenant :
- un réservoir (3) de produit (P) doté d'un col (9) délimitant une ouverture (11),
- une membrane (5) fermant l'ouverture (11) et comportant ainsi un côté interne (13) orienté vers le réservoir (3) et un côté externe (15) opposé au côté interne (13),
- une bague de fixation (7) de la membrane (5) sur le col (9), **caractérisé en ce que**
la membrane (5) étant bi-stable **en ce qu'**elle est configurée pour basculer d'une première position stable, dans laquelle la membrane (5) est bombée dans un premier sens de telle sorte qu'elle fait saillie à l'intérieur du réservoir (3) et obture le réservoir (3), à une deuxième position stable, dans laquelle la membrane (5) est bombée dans un deuxième sens opposé au premier sens de façon à être percée par une aiguille (8) disposée du côté externe (15) de la membrane (5).

2. Dispositif de distribution (1, 1') selon la revendication précédente, dans lequel la membrane (5) est en matériau élastomère, de préférence à base de bromobutyl ou de chlorobutyl.

3. Dispositif de distribution (1, 1') selon l'une quelconque des revendications précédentes, lequel comporte l'aiguille (8) en tant qu'aiguille de connexion à une tubulure de distribution (21) de produit, l'aiguille (8) étant configurée pour être distante de la membrane (5) lorsque la membrane (5) est dans la première position stable et pour traverser la membrane (5) lorsque la membrane (5) est dans la deuxième position stable.

4. Dispositif de distribution (1, 1') selon l'une quelconque des revendications précédentes, dans lequel le dispositif comporte l'aiguille (8) qui est montée fixe par rapport au réservoir (3).

5. Dispositif de distribution (1, 1') selon l'une quelconque des revendications précédentes, dans lequel la membrane (5) présente une partie périphérique (19) qui est comprimée axialement entre la bague de fixation (7) et le col (9) du réservoir (3).

6. Dispositif de distribution (1, 1') selon l'une quelconque des revendications précédentes, dans lequel la membrane (5) comprend une partie centrale (17) déformable entre la première position stable et la deuxième position stable, la partie centrale (17) présentant une épaisseur entre 0,3 et 2,5 mm, de préférence égale à 1 mm.

7. Dispositif de distribution (1, 1') selon l'une quelconque des revendications précédentes, dans lequel la membrane (5) est configurée pour basculer d'une première position stable à une deuxième position stable lorsque la pression différentielle entre le côté interne (13) et le côté externe (15) de la membrane (5) dépasse un seuil prédéterminé.

8. Dispositif de distribution (1, 1') selon la revendication précédente, dans lequel le seuil prédéterminé est entre 0,4 bar et 3 bar, de préférence entre 0,6 bar et 0,7 bar.

9. Dispositif de distribution (1, 1') selon l'une quelconque des revendications précédentes, lequel comporte une pompe (31) et un moteur électrique (33) d'entraînement de la pompe (31), la pompe (31) étant configurée pour faire basculer la membrane (5) de la première position stable dans la deuxième position stable et pour distribuer du produit (P) hors du réservoir (3) à travers l'aiguille (8) lorsque la membrane (5) est dans la deuxième position stable.

10. Dispositif de distribution (1, 1') selon l'une quelconque des revendications précédentes, lequel comporte un système de détermination de la position de la membrane (5).

11. Dispositif de distribution (1, 1') selon la revendication précédente dans sa dépendance à la revendication 9, dans lequel le système de détermination de la position de la membrane (5) comprend un organe de mesure (39) de l'intensité ou du couple du moteur électrique (33) entraînant la pompe (31).

12. Dispositif de distribution (1, 1') selon la revendication 10 ou 11, dans lequel le système de détermination de la position de la membrane (5) comprend un capteur de pression (41) configuré pour mesurer une pression du côté interne (13) et/ou du côté externe (15) de la membrane (5), de préférence configuré pour mesurer une pression différentielle entre le côté interne (13) et le côté externe (15) de la membrane (5).

13. Système de distribution d'un produit (P) comprenant un dispositif de distribution (1, 1') selon l'une quelconque des revendications précédentes et une unité de distribution de produit.

14. Kit d'assemblage pour un système de distribution d'un produit, comprenant un dispositif de distribution (1, 1') selon l'une quelconque des revendications 1 à 12 et une unité de distribution de produit.

## Patentansprüche

1. Ausgabevorrichtung (1, 1') zur Ausgabe eines Produkts (P), umfassend:
- einen Behälter (3) für das Produkt (P), der mit einem Hals (9) versehen ist, der eine Öffnung (11) begrenzt,
- eine Membran (5), die die Öffnung (11) verschließt und so eine Innenseite (13), die zu dem Behälter (3) hin weist, und eine Außenseite (15), die der Innenseite (13) gegenüberliegt, aufweist,
- einen Befestigungsring (7) zum Befestigen der Membran (5) auf dem Hals (9),
**dadurch gekennzeichnet, dass**
die Membran (5) bistabil ist, indem sie ausgebildet ist, um sich umzustülpen
aus einer ersten stabilen Position, in der die Membran (5) in einer ersten Richtung gewölbt ist, so dass sie in das Innere des Behälters (3) hineinragt und den Behälter (3) abdichtet, in eine zweite stabile Position, in der die Membran (5) in einer zweiten Richtung, die der ersten Richtung entgegengesetzt ist, gewölbt ist, so dass sie durch eine Nadel (8), die auf der Außenseite (15) der Membran (5) angeordnet ist, durchstochen wird.

2. Ausgabevorrichtung (1, 1') nach dem vorhergehenden Anspruch, wobei die Membran (5) aus Elastomermaterial, bevorzugt auf Bromobutyl- oder Chlorobutyl-Basis ist.

3. Ausgabevorrichtung (1, 1') nach einem der vorhergehenden Ansprüche, die die Nadel (8) als Nadel für die Verbindung zu einer Ausgabedüse (21) zur Ausgabe eines Produkts (P) aufweist, wobei die Nadel (8) ausgebildet ist, um von der Membran (5) entfernt zu sein, wenn die Membran (5) in der ersten stabilen Position ist, und um die Membran (5) zu durchqueren, wenn die Membran (5) in der zweiten stabilen Position ist.

4. Ausgabevorrichtung (1, 1') nach einem der vorhergehenden Ansprüche, wobei die Vorrichtung die Nadel (8) aufweist, die relativ zu dem Behälter (3) fest montiert ist.

5. Ausgabevorrichtung (1, 1') nach einem der vorhergehenden Ansprüche, wobei die Membran (5) einen Umfangsabschnitt (19) aufweist, der zwischen dem Befestigungsring (7) und dem Hals (9) des Behälters (3) axial zusammengedrückt ist.

6. Ausgabevorrichtung (1, 1') nach einem der vorhergehenden Ansprüche, wobei die Membran (5) einen mittleren Abschnitt (17) umfasst, der zwischen der ersten stabilen Position und der zweiten stabilen Position verformbar ist, wobei der mittlere Abschnitt (17) eine Dicke zwischen 0,3 mm und 2,5 mm, vorzugsweise gleich 1 mm aufweist.

7. Ausgabevorrichtung (1, 1') nach einem der vorhergehenden Ansprüche, wobei die Membran (5) ausgebildet ist, um sich aus einer ersten stabilen Position in eine zweite stabile Position umzustülpen, wenn die Druckdifferenz zwischen der Innenseite (13) und der Außenseite (15) der Membran (5) einen vorher festgelegten Schwellwert überschreitet.

8. Ausgabevorrichtung (1, 1') nach dem vorhergehenden Anspruch, wobei der vorher festgelegte Schwellwert zwischen 0,4 bar und 3 bar, bevorzugt zwischen 0,6 bar und 0,7 bar beträgt.

9. Ausgabevorrichtung (1, 1') nach einem der vorhergehenden Ansprüche, die eine Pumpe (31) und einen Elektromotor (33) zum Antreiben der Pumpe (31) aufweist, wobei die Pumpe (31) ausgebildet ist, um die Membran (5) aus der ersten stabilen Position in die zweite stabile Position umzustülpen und um von dem Produkt (P) aus dem Behälter (3) durch die Nadel (8) hindurch auszugeben, wenn die Membran (5) in der zweiten stabilen Position ist.

10. Ausgabevorrichtung (1, 1') nach einem der vorhergehenden Ansprüche, die ein System zum Bestimmen der Position der Membran (5) aufweist.

11. Ausgabevorrichtung (1, 1') nach dem vorhergehenden Anspruch in Abhängigkeit von Anspruch 9, wobei das System zum Bestimmen der Position der Membran (5) ein Messorgan (39) zum Messen der Stärke oder des Drehmoments des die Pumpe (31) antreibenden Elektromotors (33) umfasst.

12. Ausgabevorrichtung (1, 1') nach Anspruch 10 oder 11, wobei das System zum Bestimmen der Position der Membran (5) einen Druckgeber (41) umfasst, der ausgebildet ist, um einen Druck auf der Innenseite (13) und/oder auf der Außenseite (15) der Membran (5) zu messen, der bevorzugt ausgebildet ist, um einen Druckunterschied zwischen der Innenseite (13) und der Außenseite (15) der Membran (5) zu messen.

13. System zur Ausgabe eines Produkts (P), umfassend eine Ausgabevorrichtung (1, 1') nach einem der vorhergehenden Ansprüche und eine Produktausgabeeinheit.

14. Bausatz für ein System zur Ausgabe eines Produkts, umfassend eine Ausgabevorrichtung (1, 1') nach einem der vorhergehenden Ansprüche 1 bis 12 und eine Produktausgabeeinheit.

## Claims

1. Dispensing device (1, 1') for dispensing a product (P), comprising:
- a reservoir (3) of product (P) provided with a collar (9) delimiting an opening (11),
- a membrane (5) closing the opening (11) and thus having an inner side (13) oriented towards the reservoir (3) and an outer side (15) away from the inner side (13),
- a securing ring (7) for securing the membrane (5) to the collar (9), **characterized in that** the membrane (5) being bistable **in that** it is configured to flip from a first stable position, in which the membrane (5) bulges in a first direction such that it protrudes into the reservoir (3) and closes off the reservoir (3), into a second stable position, in which the membrane (5) bulges in a second direction opposite to the first direction so as to be pierced by a needle (8) disposed on the outer side (15) of the membrane (5).

2. Dispensing device (1, 1') according to the preceding claim, wherein the membrane (5) is made of elastomer material, preferably bromobutyl-based or chlorobutyl-based.

3. Dispensing device (1, 1') according to any one of the preceding claims, which comprises the needle (8) as needle for connecting to a product distribution tubing (21), the needle (8) being configured to be distant from the membrane (5) when the membrane (5) is in the first stable position and to cross through the membrane (5) when the membrane (5) is in the second stable position.

4. Dispensing device (1, 1') according to any one of the preceding claims, wherein the device comprises the needle (8) which is fixedly mounted with respect to the reservoir (3).

5. Dispensing device (1, 1') according to any one of the preceding claims, wherein the membrane (5) has a peripheral part (19) which is compressed axially between the securing ring (7) and the collar (9) of the reservoir (3).

6. Dispensing device (1, 1') according to any one of the preceding claims, wherein the membrane (5) comprises a central part (17) that is deformable between the first stable position and the second stable position, the thickness of the central part (17) being between 0.3 mm and 2.5 mm, preferably equal to 1 mm.

7. Dispensing device (1, 1') according to any one of the preceding claims, wherein the membrane (5) is configured to flip from a first stable position into a second stable position when the differential pressure between the inner side (13) and the outer side (15) of the membrane (5) is greater than a predetermined threshold.

8. Dispensing device (1, 1') according to the preceding claim, wherein the predetermined threshold is between 0.4 bar and 3 bar, preferably between 0.6 bar and 0.7 bar.

9. Dispensing device (1, 1') according to any one of the preceding claims, which comprises a pump (31) and an electric motor (33) to drive the pump (31), the pump (31) being configured to flip the membrane (5) from the first stable position into the second stable position and to dispense product (P) out of the reservoir (3) via the needle (8) when the membrane (5) is in the second stable position.

10. Dispensing device (1, 1') according to any one of the preceding claims, which comprises a system for determining the position of the membrane (5).

11. Dispensing device (1, 1') according to the preceding claim in its dependence on claim 9, wherein the system for determining the position of the membrane (5) comprises a member (39) for measuring the current or torque of the electric motor (33) driving the pump (31).

12. Dispensing device (1, 1') according to claim 10 or 11, wherein the system for determining the position of the membrane (5) comprises a pressure sensor (41) configured to measure a pressure on the inner side (13) and/or the outer side (15) of the membrane (5), preferably configured to measure a differential pressure between the inner side (13) and the outer side (15) of the membrane (5).

13. System for dispensing a product (P) comprising a dispensing device (1, 1') according to any one of the preceding claims and a product dispensing unit.

14. Assembly kit for a system for dispensing a product, comprising a dispensing device (1, 1') according to any one of claims 1 to 12 and a product dispensing unit.
